(19)

Europäisches
Patentamt

European
Patent Office

Office européen
des brevets

(11) **EP 3 135 770 A1**

(12) **EUROPEAN PATENT APPLICATION**

(43) Date of publication:
**01.03.2017 Bulletin 2017/09**

(51) Int Cl.:
***C12Q 1/68*** (2006.01)

(21) Application number: **15182851.4**

(22) Date of filing: **28.08.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(71) Applicant: **Latvian Biomedical Research and
Study Centre
1067 Riga (LV)**

(72) Inventors:
• **Radovica-Spalvina, Ilze**
**1067 Riga (LV)**

• **Rovite, Vita**
**1067 Riga (LV)**
• **Peculis, Raitis**
**1067 Riga (LV)**
• **Nikitina-Zake, Liene**
**1067 Riga (LV)**
• **Fridmanis, Davides**
**1067 Riga (LV)**
• **Klovins, Janis**
**1067 Riga (LV)**

(74) Representative: **Zaboliene, Reda
METIDA Law Firm Zaboliene and Partners
Business center VERTAS
Gyneju 16
01109 Vilnius (LT)**

(54) **SET OF OLIGONUCLEOTIDES AND METHOD FOR DETECTION OF FETAL DNA FRACTION
IN MATERNAL PLASMA**

(57)      A method for calculating the fetal ccfDNA fraction in maternal plasma comprising detecting the presence of fetus specific alleles (different from maternal alleles) of composition of common single nucleotide polymorphism (SNP) in a target polynucleotide in a subject, wherein the SNP comprises rs10873704, rs2316921, rs3991951, rs13142086, rs2598548, rs10155423, rs7683293, rs11132694, rs2287678, rs888726, rs7740992, rs11242795, rs7756422, rs6915731, rs9347210, rs9691429, rs4875906, rs10780162, rs2005486, rs3911483, rs2028902, rs2904516, rs2175938, rs1973118, rs6498782, rs3826238, rs11568105, rs2139261, rs4494584 and rs55969137, and wherein detecting the presence of the fetal specific alleles (different from maternal alleles) of SNP in the subject is indicative of the fetal ccfDNA fraction in maternal plasma.

**Description**

**Field of the invention**

The present invention relates to a composition of common single nucleotide polymorphisms and methods for detection of fetal circulating cell free DNA (ccfDNA) fraction in maternal plasma.

**Background of the invention**

Prenatal diagnosis is testing for diseases or conditions in a fetus or embryo before it is born. The aim is to detect birth defects such as neural tube defects, Down syndrome, chromosome abnormalities, genetic disorders and other conditions. Common testing procedures include amniocentesis, ultrasonography including nuchal translucency ultrasound, serum marker testing, or genetic screening. In some cases, the tests are administered to determine if the fetus will be aborted, though physicians and patients also find it useful to diagnose high-risk pregnancies early so that delivery can be scheduled in a tertiary care hospital where the baby can receive appropriate care.

Amniocentesis carries a miscarriage risk of 1 in 300 to 1 in 500 [1] and a small risk of other complications [2, 3]. Chorionic villus sampling carries a similar miscarriage risk and a 1/3,000 risk of fetal limb reduction defects, especially when performed before 10 weeks of gestation [4, 5]. The identification of fetal circulating cell-free DNA (ccfDNA) in maternal plasma facilitated the development of noninvasive prenatal screening methods for fetal whole chromosomal aneuploidies with improved detection rates and accuracies as compared with traditional screening methods [6-19]. The most straightforward ccfDNA based approaches use nonspecific amplification followed by massively parallel shotgun sequencing also known as next generation sequencing (NGS), identifying abnormal amounts of DNA from chromosome of interest relative to reference chromosomes [8-10, 14-16, 18]. The use of NGS methods to identify trisomy depends on the ability to differentiate a 50% increase in the amount of the fetal chromosome of interest in the face of a large proportion of euploid maternal ccfDNA [20]. The problem with this approach is that there is a much larger amount of maternal-derived ccfDNA than fetal-derived ccfDNA, making the excess of trisomy affected chromosome sequences very small [21]. The accuracy of ccfDNA testing depends on the proportion of the fetal to maternal origin of ccfDNA in maternal blood and the minimum required fetal fraction (FF) for aneuploidy assessment with current methods is 4%; a lower FF could lead to a false negative result [9, 10, 16, 18].

There are different approaches used to detect the amount of fetal ccfDNA fraction including very simple methods such as Y chromosome qualitative and quantitative detection using real-time PCR [21], and much advanced technologies for example ccfDNA size based approach [22] or even using methylation differences between maternal and fetal ccfDNA [23].

Targeted resequencing of common single nucleotide polymorphisms (SNPs) in high coverage is also a way to determine fetal ccfDNA fraction [24]. However, it is well known in the art that genetic variation exists among human individuals and in particular between ethnic groups. There are marked physical and physiological differences among human populations that presumably reflect long-term adaptation to unique ecological conditions, random genetic drift, and sex selection. There are thus genetic markers that exist in one population and not in others and there are also genetic markers at loci at which different alleles are fixed in different populations. Accordingly, the current method of systemic screening is not universal and equally accurate for all populations.

Patent US20100376637P provides methods for detecting the presence of fetal DNA in a biological sample of a maternal host. Specifically, the method comprises identifying the genotype of at least one conserved genomic segment based on the genomic segment information provided. The disclosure further provides genetic markers in chromosomal locations associated with fetal abnormalities for detecting a genetic condition of a fetus using a biological sample of a maternal host. The method for enrichment of cell free fetal DNA from maternal whole blood sample by DNA size fractionation is also described.

Patent US201261604984P provides methods and materials useful for detecting fetal DNA as well as markers for fetal conditions by using biological samples of a maternal host.

Patent AU20130903743 relates generally to an assay to detect and quantitate target nucleic acid in a mixture of target and non-target nucleic acid, kits useful for same and its use in non-invasive diagnostic methodologies. The assay comprises identifying copy number variation (CNV) polymorphisms present in the target nucleic acid and absent in non-target nucleic acid. Embodiments disclosed include quantitating the level of fetal DNA in sample of maternal DNA and fetal DNA, and quantitating the level of donor-derived DNA in sample from a transplant recipient comprising self DNA and non-self or donor DNA.

Patent US201161474362P Methods of reliably estimating genomic fraction (e.g., fetal fraction) from polymorphisms such as small base variations or insertions-deletions are disclosed. Sequenced data from a multigenomic source is used to determine allele counts for one or more of the polymorphisms. For one or more of the polymorphisms, zygosity is assigned, and genomic fraction is determined from the zygosity and allele counts. Certain embodiments employ SNPs as the relevant polymorphism. The disclosed methods can be applied as part of an intentional, pre-designed re-sequencing study targeted against known polymorphisms or can be used in a retrospective analysis of variations found by coincidence in overlapping sequences generated from maternal plasma.

Patent ES20100830939T The invention provides compositions and methods for simultaneously determining the presence or absence of fetal aneuploidy and the relative amount of fetal nucleic acids in a sample obtained from a pregnant female. The method encompasses the use of sequencing technologies and exploits the occurrence of polymorphisms to provide a streamlined noninvasive process applicable to the practice of prenatal diagnostics.

However these inventions are quite different as they focus on genetic markers associated with abnormalities to test the amount and risk for certain conditions in fetus from circulating cell free DNA and use different set of polymorphisms.

Aim of the invention is to provide a method for detection of ccfDNA fraction in maternal plasma.

## Summary of the invention

Present invention includes a method for detection of ccfDNA fraction in maternal plasma comprising detecting the presence of fetus specific alleles (different from maternal alleles) of specific single nucleotide polymorphisms (SNPs) in a target polynucleotide in a subject. The SNPs comprises rs10873704 (SEQ IN NO. 1), rs2316921 (SEQ IN NO. 2), rs3991951 (SEQ IN NO. 3), rs13142086 (SEQ IN NO. 4), rs2598548 (SEQ IN NO. 5), rs10155423 (SEQ IN NO. 6), rs7683293 (SEQ IN NO. 7), rs11132694 (SEQ IN NO. 8), rs2287678 (SEQ IN NO. 9), rs888726 (SEQ IN NO. 10), rs7740992 (SEQ IN NO. 11), rs11242795 (SEQ IN NO. 12), rs7756422 (SEQ IN NO. 13), rs6915731 (SEQ IN NO. 14), rs9347210 (SEQ IN NO. 15), rs9691429 (SEQ IN NO. 16), rs4875906 (SEQ IN NO. 17), rs10780162 (SEQ IN NO. 18), rs2005486 (SEQ IN NO. 19), rs3911483 (SEQ IN NO. 20), rs2028902 (SEQ IN NO. 21), rs2904516 (SEQ IN NO. 22), rs2175938 (SEQ IN NO. 23), rs1973118 (SEQ IN NO. 24), rs6498782 (SEQ IN NO. 25), rs3826238 (SEQ IN NO. 26), rs11568105 (SEQ IN NO. 27), rs2139261 (SEQ IN NO. 28), rs4494584 (SEQ IN NO. 29) and/or rs55969137 (SEQ IN NO. 30). Detecting a presence of fetal allele of the SNPs in the subject is indicative for calculating fetal derived ccfDNA fraction in maternal plasma.

Said invention also includes detecting the presence of a single nucleotide polymorphism (SNP) at a position rs10873704 (nucleotide 26 on SEQ IN NO. 1) or any of rs2316921 (nucleotide 26 on SEQ IN NO. 2), rs3991951 (nucleotide 26 on SEQ IN NO. 3), rs13142086 (nucleotide 26 on SEQ IN NO. 4), rs2598548 (nucleotide 26 on SEQ IN NO. 5), rs10155423 (nucleotide 26 on SEQ IN NO. 6), rs7683293 (nucleotide 26 on SEQ IN NO. 7), rs11132694 (nucleotide 26 on SEQ IN NO. 8), rs2287678 (nucleotide 26 on SEQ IN NO. 9), rs888726 (nucleotide 26 on SEQ IN NO. 10), rs7740992 (nucleotide 26 on SEQ IN NO. 11), rs11242795 (nucleotide 26 on SEQ IN NO. 12), rs7756422 (nucleotide 26 on SEQ IN NO. 13), rs6915731 (nucleotide 26 on SEQ IN NO. 14), rs9347210 (nucleotide 26 on SEQ IN NO. 15), rs9691429 (nucleotide 26 on SEQ IN NO. 16), rs4875906 (nucleotide 26 on SEQ IN NO. 17), rs10780162 (nucleotide 26 on SEQ IN NO. 18), rs2005486 (nucleotide 26 on SEQ IN NO. 19), rs3911483 (nucleotide 26 on SEQ IN NO. 20), rs2028902 (nucleotide 26 on SEQ IN NO. 21), rs2904516 (nucleotide 26 on SEQ IN NO. 22), rs2175938 (nucleotide 26 on SEQ IN NO. 23), rs1973118 (nucleotide 26 on SEQ IN NO. 24), rs6498782 (nucleotide 26 on SEQ IN NO. 25), rs3826238 (nucleotide 26 on SEQ IN NO. 26), rs11568105 (nucleotide 26 on SEQ IN NO. 27), rs2139261 (nucleotide 26 on SEQ IN NO. 28), rs4494584 (nucleotide 26 on SEQ IN NO. 29) or rs55969137 (nucleotide 26 on SEQ IN NO. 30).

A probe for the fetal ccfDNA fraction detection in maternal plasma comprising composition of single nucleotide polymorphism (SNP) set forth as rs10873704 (SEQ IN NO. 1), rs2316921 (SEQ IN NO. 2), rs3991951 (SEQ IN NO. 3), rs13142086 (SEQ IN NO. 4), rs2598548 (SEQ IN NO. 5), rs10155423 (SEQ IN NO. 6), rs7683293 (SEQ IN NO. 7), rs11132694 (SEQ IN NO. 8), rs2287678 (SEQ IN NO. 9), rs888726 (SEQ IN NO. 10), rs7740992 (SEQ IN NO. 11), rs11242795 (SEQ IN NO. 12), rs7756422 (SEQ IN NO. 13), rs6915731 (SEQ IN NO. 14), rs9347210 (SEQ IN NO. 15), rs9691429 (SEQ IN NO. 16), rs4875906 (SEQ IN NO. 17), rs10780162 (SEQ IN NO. 18), rs2005486 (SEQ IN NO. 19), rs3911483 (SEQ IN NO. 20), rs2028902 (SEQ IN NO. 21), rs2904516 (SEQ IN NO. 22), rs2175938 (SEQ IN NO. 23), rs1973118 (SEQ IN NO. 24), rs6498782 (SEQ IN NO. 25), rs3826238 (SEQ IN NO. 26), rs11568105 (SEQ IN NO. 27), rs2139261 (SEQ IN NO. 28), rs4494584 (SEQ IN NO. 29) and/or rs55969137 (SEQ IN NO. 30). Said probes can be implemented also as a kit including additional elements for example fluorescents for ease of detection of said SNPs. In another embodiment a probe can comprise composition of SNPs set for the as rs10873704 (nucleotide 26 on SEQ IN NO. 1) or any of rs2316921 (nucleotide 26 on SEQ IN NO. 2), rs3991951 (nucleotide 26 on SEQ IN NO. 3), rs13142086 (nucleotide 26 on SEQ IN NO. 4), rs2598548 (nucleotide 26 on SEQ IN NO. 5), rs10155423 (nucleotide 26 on SEQ IN NO. 6), rs7683293 (nucleotide 26 on SEQ IN NO. 7), rs11132694 (nucleotide 26 on SEQ IN NO. 8), rs2287678 (nucleotide 26 on SEQ IN NO. 9), rs888726 (nucleotide 26 on SEQ IN NO. 10), rs7740992 (nucleotide 26 on SEQ IN NO. 11), rs11242795 (nucleotide 26 on SEQ IN NO. 12), rs7756422 (nucleotide 26 on SEQ IN NO. 13), rs6915731 (nucleotide 26 on SEQ IN NO. 14), rs9347210 (nucleotide 26 on SEQ IN NO. 15), rs9691429 (nucleotide 26 on SEQ IN NO. 16), rs4875906 (nucleotide 26 on SEQ IN NO. 17), rs10780162 (nucleotide 26 on SEQ IN NO. 18), rs2005486 (nucleotide 26 on SEQ IN NO. 19), rs3911483 (nucleotide 26 on SEQ IN NO. 20), rs2028902 (nucleotide 26 on SEQ IN NO. 21), rs2904516 (nucleotide 26 on SEQ IN NO. 22), rs2175938 (nucleotide 26 on SEQ IN NO. 23), rs1973118 (nucleotide 26 on SEQ IN NO. 24), rs6498782 (nucleotide 26 on SEQ IN NO. 25), rs3826238 (nucleotide 26 on SEQ IN NO. 26), rs11568105 (nucleotide 26 on SEQ IN NO. 27), rs2139261 (nucleotide 26 on SEQ IN NO. 28), rs4494584 (nucleotide 26 on SEQ IN NO. 29) or rs55969137 (nucleotide 26 on SEQ IN NO. 30).

According to one embodiment, the present invention relates to a method for detecting the fraction of fetal derived ccfDNA in maternal plasma in an individual, the method comprise detecting fetal specific allele (different from maternal alleles) in specific genetic variation pattern (SNPs pattern). In particular, the SNPs are selected from the group of loci with nucleotide base change consisting of: CHR1, g.85980349 A>G (SEQ ID NO. 1), CHR1, g.104626818 A>G (SEQ ID NO. 2), CHR1, g.224216756 A>G (SEQ ID NO. 3), CHR4, g.126503918 A>T (SEQ ID NO. 4), CHR4, g.137069205 A>G (SEQ ID NO. 5), CHR4, g.170281121 C>G (SEQ ID NO. 6), CHR4, g.174342567 G>T (SEQ ID NO. 7), CHR4, g.190540979 A>T (SEQ ID NO. 8), CHR5, g.102443043 C>T (SEQ ID NO. 9), CHR5, g.175348069 A>G (SEQ ID NO. 10), CHR6, g.261390 A>G (SEQ ID NO. 11), CHR6, g.322255 A>G (SEQ ID NO. 12), CHR6, g.57268764 G>T (SEQ ID NO. 13), CHR6, g.146863404 A>G (SEQ ID NO. 14), CHR6, g.167774024 A>G (SEQ ID NO. 15), CHR7, g.61915623 A>G (SEQ ID NO. 16), CHR8, g.2205101 C>T (SEQ ID NO. 17), CHR8, g.2275622 A>G (SEQ ID NO. 18), CHR8, g.58122242 C>T (SEQ ID NO. 19), CHR8, g.110427192 C>T (SEQ ID NO. 20), CHR11, g.36309909 A>T (SEQ ID NO. 21), CHR12, g.66996166 A>G (SEQ ID NO. 22), CHR15, g.22428943 C>T (SEQ ID NO. 23), CHR15, g.51645910 A>T (SEQ ID NO. 24), CHR16, g.18606396 A>G (SEQ ID NO. 25), CHR16, g.70182024 A>G (SEQ ID NO. 26), CHR17, g.6562461 A>G (SEQ ID NO. 27), CHR17, g.21313222 C>G (SEQ ID NO. 28), CHR17, g.72786911 A>T (SEQ ID NO. 29), CHR22, g.50899756 A>C>G>T (SEQ ID NO. 30), where "g" stands for genomic sequence and "CHR" stands for number of chromosome. Even more in particular, the SNPs may be selected from the variations listed in TABLE 1.

TABLE 1

| List of SNPs | | | |
|---|---|---|---|
| SNP | rs number | Sequence | SEQ ID NO. |
| CHR 1, g.85980349A>G | rs10873704 | CTTTTCTTTCACTTTTTTGGGTATT[A/G]CTATGG ACTAATGAATTTTTATGTA | 1 |
| CHR1, g.104626818A>G | rs2316921 | AGATAGATAGGCAGCCAACAAAAGT[A/G]TACA TACACACACAGTGAATAAGCA | 2 |
| CHR1, g.224216756A>G | rs3991951 | ACAATGCAGCCCTTTTGCTTCCTAA[A/G]CTCAC CAGTTCCCTCGCTCCACCTC | 3 |
| CHR4, g.126503918A>T | rs13142086 | CTTATTTCAGTTTAGTGCAGATGAA[A/T]TTTTTT TTTTTTTTTATGGAGGTCA | 4 |
| CHR4, g.137069205A>G | rs2598548 | GCAGGTGGGTAGTTGAAGTGTGTGT[A/G]TGTGT GTGTGTGTGTTTTATTTCCT | 5 |
| CHR4, g.170281121C>G | rs10155423 | AAGCAAAAAGAAAACCAATCAAAAC[C/G]CTAC ACTTTAACTTTGTTCCCCTGA | 6 |
| CHR4, g.174342567G>T | rs7683293 | TTTCTCTGTTTTGGTTTAGTTTGTT[G/T]GGGGGG GGTTGATTTGTTGCATTTT | 7 |
| CHR4, g.190540979A>T | rs11132694 | CAAGAGGCTTCCAGTGCTCCCCAGA[A/T]GGGTT TTGACATCTTATCATTGTTT | 8 |
| CHR5, g.102443043C>T | rs2287678 | AAAATAATGTTTTATCATCACTGTA[C/T]TTTTGC AAGCAAACAGCTCTCTCAG | 9 |
| CHR5, g.175348069A>G | rs888726 | AGATTGACCTTACATGGTTGCCTGG[A/G]AGATC CAATGAGATCAGTACGAACA | 10 |
| CHR6, g.261390A>G | rs7740992 | TCAAAATATCTCATGAGAGTGGTGC[A/G]TACAA TTAATGAACCTACCTTGACA | 11 |

(continued)

| | | List of SNPs | |
|---|---|---|---|
| **SNP** | **rs number** | **Sequence** | **SEQ ID NO.** |
| CHR6, g.322255A>G | rs11242795 | GGGAATGATGGTGGGGCCATCCTCC[A/G]GCGTC TTGCTTTTATGTGGTGTTAT | 12 |
| CHR6, g.57268764G>T | rs7756422 | GGTTTGCAGATGGCTGTCTTCTCAT[G/T]GTATCT TCAAATGGTTGAGAGCAGA | 13 |
| CHR6, g.146863404A>G | rs6915731 | CCACCACTGTTTCCACGTATTCCGT[A/G]TGTGTG TGTGTGTGTGTGTGTGTGT | 14 |
| CHR6, g.167774024A>G | rs9347210 | TAAATTTATATTTCCAAACCAGGAC[A/G]CTTGT GTCACTTAGCTGCTTAAACC | 15 |
| CHR7, g.61915623A>G | rs9691429 | TGAGAAAATTCTTTGTGACGAGTGC[A/G]TTCAA CTCACAGAGTTAAATTTCTT | 16 |
| CHR8, g.2205101C>T | rs4875906 | AAATCAAGCATGGTCATAACTGTTT[C/T]AGCAG CATTTTTCAGTCGTGCGTAC | 17 |
| CHR8, g.2275622A>G | rs10780162 | TCTTGGTGAATACTCCATTCGACAC[A/G]TTAAA AGAATGTGTATTCTTTTGTT | 18 |
| CHR8, g.58122242C>T | rs2005486 | AAGGGAGAAATTTCCAGCCCCCTTG[C/T]GGCAG CTAGCTGAAAAACAGGCTCC | 19 |
| CHR8, g.110427192C>T | rs3911483 | CCTGTTTTCTACATCATCCCAACAT[C/T]ATTATT ATTATTATTATTATTATTA | 20 |
| CHR 11, g.36309909A>T | rs2028902 | ACTCAACCCTCTTCACCTGGTGTTT[A/T]AAAAA AAAAAAAAAGAGAGAGGAAG | 21 |
| CHR12, g.66996166A>G | rs2904516 | TAAAATGATGTGTAACTACAATCAC[A/G]GGGGC CCCGCATAATTTAAAGAAAT | 22 |
| CHR15, g.22428943C>T | rs2175938 | TCTCATGCCTCCTGGTGTCTCTGGG[C/T]GATCAA TGTGGCCTTAAGATGCTCG | 23 |
| CHR15, g.51645910A>T | rs1973118 | TGTGGTTTTTTTTTTTTTTTTTTT[A/T]ATCAAAT CCGAGGACTGTTCTGATA | 24 |
| CHR16, g.18606396A>G | rs6498782 | AAGACTTGCTAAGTGGAGAATTTCT[A/G]TGTCC AGTCATTCCTGGATCACTTC | 25 |
| CHR16, g.70182024A>G | rs3826238 | GTAGCAGGCACAATGAGGACAAGGC[A/G]AGTG TCTTCTCTGGGGGAATCAGCA | 26 |
| CHR17, g.6562461A>G | rs11568105 | CAAGATGGAATGGTTGATAGAAACA[A/G]GGTC AAATGAAGAAATGTGACTGGC | 27 |
| CHR17, g.21313222C>G | rs2139261 | AGAGACTCAGCTGACCTCTGTCCAC[C/G]AAGCT AACCCATGGCCCTCAAACAT | 28 |
| CHR17, g.72786911A>T | rs4494584 | AGCAGGAGCATCTCTGAATTCCCTT[A/T]AAAAA AAAAAAAAAAAAAGACTGT | 29 |
| CHR22, g.50899756A>C>G>T | rs55969137 | CAGAGTCAGAAGCAAGGAAGGTAAG[A/C/G/T]G GGGGGGGGTCCCAGAATCTCAGGG | 30 |

The present invention also provides microarrays, chips, and/or kits comprising at least one probe capable of hybridizing to at least one SNP according to any aspect of the present invention.

**Detailed description of the preferred embodiments**

Definitions for convenience, certain terms employed in the specification, examples and appended claims are collected here.

The term "comprising" is herein defined to be that where the various components, ingredients, or steps, can be conjointly employed in practicing the present invention. Accordingly, the term "comprising" encompasses the more restrictive terms "consisting essentially of" and "consisting of." With the term "consisting essentially of" it is understood that the method according to any aspect of the present invention "substantially" comprises the indicated SNP as "essential" element. Additional SNPs may be included. Accordingly, a method "consisting essentially of" a plurality of SNPs will be novel in view of a known polypeptide accidentally comprising one of the SNPs. With the term "consisting of" it is understood that the method, microarray and/or chip according to the invention corresponds to all of the SNPs.

The term "label" or "label containing moiety" refers in a moiety capable of detection, such as a radioactive isotope or group containing same and nonisotopic labels, such as enzymes, biotin, avidin, streptavidin, digoxygenin, luminescent agents, dyes, haptens, and the like. Luminescent agents, depending upon the source of exciting energy, can be classified as radio luminescent, chemiluminescent, bio luminescent, and photo luminescent (including fluorescent and phosphorescent). A probe described herein can be bound, for example, chemically bound to label-containing moieties or can be suitable to be so bound. The probe can be directly or indirectly labelled.

The term "polymorphism" is herein defined to be the occurrence of genetic variations that account for alternative DNA sequences and/or alleles among individuals in a population.

The term "probe" is herein defined to be an oligonucleotide. A probe can be single stranded at the time of hybridization to a target. Probes include but are not limited to primers, i.e., oligonucleotides that can be used to prime a reaction, for example at least in a PCR reaction. The term "reference nucleotide sequence" used interchangeably with the term "reference sequence" is herein defined to be for a nucleotide sequence of a particular gene for example, in NCBI databases.

Alleles that differ from the reference are referred to as "variant" alleles. The polypeptide encoded by the reference nucleotide sequence is the "reference" polypeptide with a particular reference amino acid sequence, and polypeptides encoded by variant alleles are referred to as "variant" polypeptides with variant amino acid sequences. Nucleotide sequence variants can result in changes affecting properties of a polypeptide. These sequence differences, when compared to a reference nucleotide sequence, include insertions, deletions, conversions and substitutions: e.g. an insertion, a deletion or a conversion may result in a frame shift generating an altered polypeptide; a substitution of at least one nucleotide may result in a premature stop codon, amino acid change or abnormal mRNA splicing; the deletion of several nucleotides, resulting in a deletion of one or more amino acids encoded by the nucleotides; the insertion of several nucleotides, such as by unequal recombination or gene conversion, resulting in an interruption of the coding sequence of a reading frame; duplication of all or a part of a sequence; transposition; or a rearrangement of a nucleotide sequence.

The term "allele" is herein defined to one of a number of alternative forms of the same gene or same genetic locus. It is the alternative form of a gene for a character producing different effects. Sometimes, different alleles can result in different phenotypic traits.

The term "sample" is herein defined to include being blood plasma from pregnant individual.

Specific polymorphisms may be used to determine the fraction of fetal ccfDNA in maternal plasma. In particular, these polymorphisms may be SNPs. More in particular, out of these hundreds of SNPs known in the art at least 30 may be related to determination of fetal ccfDNA fraction in maternal plasma.

There is thus a need to provide a method to determine the fetal ccfDNA fraction in maternal plasma. In particular, there is a need to determine the fetal specific allele (different from maternal alleles) of polymorphisms. More in particular, the SNP are selected from the group consisting of: CHR1, g.85980349 A>G (SEQ ID NO. 1), CHR1, g.104626818 A>G (SEQ ID NO. 2), CHR1, g.224216756 A>G (SEQ ID NO. 3), CHR4, g.126503918 A>T (SEQ ID NO. 4), CHR4, g.137069205 A>G (SEQ ID NO. 5), CHR4, g.170281121 C>G (SEQ ID NO. 6), CHR4, g.174342567 G>T (SEQ ID NO. 7), CHR4, g.190540979 A>T (SEQ ID NO. 8), CHR5, g.102443043 C>T (SEQ ID NO. 9), CHR5, g.175348069 A>G (SEQ ID NO. 10), CHR6, g.261390 A>G (SEQ ID NO. 11), CHR6, g.322255 A>G (SEQ ID NO. 12), CHR6, g.57268764 G>T (SEQ ID NO. 13), CHR6, g.146863404 A>G (SEQ ID NO. 14), CHR6, g.167774024 A>G (SEQ ID NO. 15), CHR7, g.61915623 A>G (SEQ ID NO. 16), CHR8, g.2205101 C>T (SEQ ID NO. 17), CHR8, g.2275622 A>G (SEQ ID NO. 18), CHR8, g.58122242 C>T (SEQ ID NO. 19), CHR8, g.110427192 C>T (SEQ ID NO. 20), CHR11, g.36309909 A>T (SEQ ID NO. 21), CHR12, g.66996166 A>G (SEQ ID NO. 22), CHR15, g.22428943 C>T (SEQ ID NO. 23), CHR15, g.51645910 A>T (SEQ ID NO. 24), CHR16, g.18606396 A>G (SEQ ID NO. 25), CHR16, g.70182024 A>G (SEQ ID NO. 26), CHR17, g.6562461 A>G (SEQ ID NO. 27), CHR17, g.21313222 C>G (SEQ ID NO. 28), CHR17, g.72786911 A>T (SEQ ID NO. 29), CHR22, g.50899756 A>C>G>T (SEQ ID NO. 30). Even more in particular, the SNPs may be selected from the SNPs listed in TABLE 1.

In particular, the method comprises detecting the presence of combination of 30 SNPs as described in a sample of the individual. In particular, the SNPs are: CHR1, g.85980349 A>G (SEQ ID NO. 1), CHR1, g.104626818 A>G (SEQ ID NO. 2), CHR1, g.224216756 A>G (SEQ ID NO. 3), CHR4, g.126503918 A>T (SEQ ID NO. 4), CHR4, g.137069205 A>G (SEQ ID NO. 5), CHR4, g.170281121 C>G (SEQ ID NO. 6), CHR4, g.174342567 G>T (SEQ ID NO. 7), CHR4, g.190540979 A>T (SEQ ID NO. 8), CHR5, g.102443043 C>T (SEQ ID NO. 9), CHR5, g.175348069 A>G (SEQ ID NO. 10), CHR6, g.261390 A>G (SEQ ID NO. 11), CHR6, g.322255 A>G (SEQ ID NO. 12), CHR6, g.57268764 G>T (SEQ ID NO. 13), CHR6, g.146863404 A>G (SEQ ID NO. 14), CHR6, g.167774024 A>G (SEQ ID NO. 15), CHR7, g.61915623 A>G (SEQ ID NO. 16), CHR8, g.2205101 C>T (SEQ ID NO. 17), CHR8, g.2275622 A>G (SEQ ID NO. 18), CHR8, g.58122242 C>T (SEQ ID NO. 19), CHR8, g.110427192 C>T (SEQ ID NO. 20), CHR11, g.36309909 A>T (SEQ ID NO. 21), CHR12, g.66996166 A>G (SEQ ID NO. 22), CHR15, g.22428943 C>T (SEQ ID NO. 23), CHR15, g.51645910 A>T (SEQ ID NO. 24), CHR16, g.18606396 A>G (SEQ ID NO. 25), CHR16, g.70182024 A>G (SEQ ID NO. 26), CHR17, g.6562461 A>G (SEQ ID NO. 27), CHR17, g.21313222 C>G (SEQ ID NO. 28), CHR17, g.72786911 A>T (SEQ ID NO. 29), CHR22, g.50899756 A>C>G>T (SEQ ID NO. 30). More in particular, the SNP are listed in Table 1.

A list of SNPs that are used to determine fetal ccfDNA fraction in maternal plasma in an individual. The method comprises detecting all SNPs as described in a sample of the individual. In the genetic context, fetal specific alleles (different from maternal alleles) of at least three SNPs, allows to detect fetal ccfDNA fraction in maternal plasma.

According to any aspect of the present invention, the method for detection of fetal ccfDNA fraction in maternal plasma in an individual, consisting of or consisting essentially of detecting the presence fetal specific alleles (different from maternal alleles) of 30 SNPs disclosed. More in particular, the method involves the detection of 30 SNPs according to any aspect of the present invention.

According to another embodiment, there is provided a microarray and/or DNA chip comprising, consisting of or consisting essentially of probes capable of hybridizing to 30 SNPs according to any aspect of the present invention in a sample nucleic acid of an individual, wherein the SNPs are selected from the group consisting of: CHR1, g.85980349 A>G (SEQ ID NO. 1), CHR1, g.104626818 A>G (SEQ ID NO. 2), CHR1, g.224216756 A>G (SEQ ID NO. 3), CHR4, g.126503918 A>T (SEQ ID NO. 4), CHR4, g.137069205 A>G (SEQ ID NO. 5), CHR4, g.170281121 C>G (SEQ ID NO. 6), CHR4, g.174342567 G>T (SEQ ID NO. 7), CHR4, g.190540979 A>T (SEQ ID NO. 8), CHR5, g.102443043 C>T (SEQ ID NO. 9), CHR5, g.175348069 A>G (SEQ ID NO. 10), CHR6, g.261390 A>G (SEQ ID NO. 11), CHR6, g.322255 A>G (SEQ ID NO. 12), CHR6, g.57268764 G>T (SEQ ID NO. 13), CHR6, g.146863404 A>G (SEQ ID NO. 14), CHR6, g.167774024 A>G (SEQ ID NO. 15), CHR7, g.61915623 A>G (SEQ ID NO. 16), CHR8, g.2205101 C>T (SEQ ID NO. 17), CHR8, g.2275622 A>G (SEQ ID NO. 18), CHR8, g.58122242 C>T (SEQ ID NO. 19), CHR8, g.110427192 C>T (SEQ ID NO. 20), CHR11, g.36309909 A>T (SEQ ID NO. 21), CHR12, g.66996166 A>G (SEQ ID NO. 22), CHR15, g.22428943 C>T (SEQ ID NO. 23), CHR15, g.51645910 A>T (SEQ ID NO. 24), CHR16, g.18606396 A>G (SEQ ID NO. 25), CHR16, g.70182024 A>G (SEQ ID NO. 26), CHR17, g.6562461 A>G (SEQ ID NO. 27), CHR17, g.21313222 C>G (SEQ ID NO. 28), CHR17, g.72786911 A>T (SEQ ID NO. 29), CHR22, g.50899756 A>C>G>T (SEQ ID NO. 30) and the like. More in particular, the SNP may be SNPs selected from TABLE 1.

The probes capable of hybridizing to all SNPs according to any aspect of the present invention in a sample nucleic acid of an individual are designed using any one of SEQ ID NO. 1- 30 in TABLE 1.

According to a further aspect of the invention, there is provided a kit for determining fetal ccfDNA fraction in maternal plasma, the kit comprising: (a) at least 30 oligonucleotides that can be used to calculate the fetal ccfDNA fraction in maternal plasma according to any aspect of the present invention wherein the SNP may be selected from the group consisting of: CHR1, g.85980349 A>G (SEQ ID NO. 1), CHR1, g.104626818 A>G (SEQ ID NO. 2), CHR1, g.224216756 A>G (SEQ ID NO. 3), CHR4, g.126503918 A>T (SEQ ID NO. 4), CHR4, g.137069205 A>G (SEQ ID NO. 5), CHR4, g.170281121 C>G (SEQ ID NO. 6), CHR4, g.174342567 G>T (SEQ ID NO. 7), CHR4, g.190540979 A>T (SEQ ID NO. 8), CHR5, g.102443043 C>T (SEQ ID NO. 9), CHR5, g.175348069 A>G (SEQ ID NO. 10), CHR6, g.261390 A>G (SEQ ID NO. 11), CHR6, g.322255 A>G (SEQ ID NO. 12), CHR6, g.57268764 G>T (SEQ ID NO. 13), CHR6, g.146863404 A>G (SEQ ID NO. 14), CHR6, g.167774024 A>G (SEQ ID NO. 15), CHR7, g.61915623 A>G (SEQ ID NO. 16), CHR8, g.2205101 C>T (SEQ ID NO. 17), CHR8, g.2275622 A>G (SEQ ID NO. 18), CHR8, g.58122242 C>T (SEQ ID NO. 19), CHR8, g.110427192 C>T (SEQ ID NO. 20), CHR11, g.36309909 A>T (SEQ ID NO. 21), CHR12, g.66996166 A>G (SEQ ID NO. 22), CHR15, g.22428943 C>T (SEQ ID NO. 23), CHR15, g.51645910 A>T (SEQ ID NO. 24), CHR16, g.18606396 A>G (SEQ ID NO. 25), CHR16, g.70182024 A>G (SEQ ID NO. 26), CHR17, g.6562461 A>G (SEQ ID NO. 27), CHR17, g.21313222 C>G (SEQ ID NO. 28), CHR17, g.72786911 A>T (SEQ ID NO. 29), CHR22, g.50899756 A>C>G>T (SEQ ID NO. 30) and the like; and (b) instructions for use. More in particular, the SNP are selected from the SNPs listed in TABLE 1.

Having now generally described the invention, the same will be more readily understood through reference to the following examples which are provided by way of illustration, and are not intended to be limiting of the present invention.

**Example**

A person skilled in the art will appreciate that the present invention may be practised without undue experimentation according to the method given herein. The methods, techniques and chemicals are as described in the references given or from protocols in standard biotechnology and molecular biology text books.

**Study population and data collection**

The study included one pregnant female subject from the Genome Database of Latvian Population (LGDB). Written informed consent was acquired from the LGDB participant before her inclusion in the register. The study protocol was approved by the Central Medical Ethics Committee of Latvia (Protocols Nr. 01-29.1/1).
Blood samples were taken in the 12-13 or 20th week of gestation using two Cell-Free DNA BCT blood collection tubes (Streck Inc, USA). In total 20ml of blood was taken.

**DNA isolation and normalization**

DNA was isolated from blood plasma using QIAamp Circulating Nucleic Acid Kit according to manufacturer's instructions. Qualitative and quantitative estimations were carried out on the DNA samples.

**Genotyping assay design**

Selection of SNPs was based on their frequency in general population (minor allele frequency about 50% according to 1000 Genome phase one genotype data from 1094 worldwide individuals http://browser.1000genomes.org). Random 30 SNPs were chosen representing different chromosomes. List of SNPs are shown in TABLE 1. Thermo Fisher scientific Ion AmpliSeq Designer was used to produce oligonucleotide sequences that will be used to amplify oligonucleotides containing chosen SNPs (listed in TABLE 1) in multiplex PCR reaction.

**DNA library preparation**

Oligonucleotides capable to amplify specific oligonucleotide regions containing chosen SNPs (listed in TABLE 1) were all mixed in one pool and used to perform standard multiplex PCR reaction. The product of multiplex PCR was purified using NucleoMag® NGS Clean-up and Size select reagents as described in manufacturer's instructions. Qualitative and quantitative estimations were carried out on the multiplex PCR reaction product using Agilent High Sensitivity DNA kit reagents and chips described in manufacturer's instructions (Agilent Technologies). The amount of multiplex PCR product needed to combine with ccfDNA is calculated using formula:

$$\text{X ng (amount of multiplex PCR needed to combine with ccfDNA)} = \frac{4200000 * \text{Y ng (amount of ccfDNA in 20}\mu\text{l)}}{66000000}$$

The total volume of the mixture should be 39 μl. It is achieved by adding appropriate volume of deionized water or if the volume of the mixture exceeds 39 μl the volume of ccfDNA should be reduced till the total volume of mixture is 39 μl
Standard molecular biology techniques known in the art and not specifically described were generally followed as described in manufacturer's instructions (Thermo Fisher Scientific, Ion Plus Fragment Library Kit) to produce DNA libraries. Qualitative and quantitative estimations were carried out on the produced DNA libraries using Agilent High Sensitivity DNA kit reagents and chips described in manufacturer's instructions (Agilent Technologies).

**NGS sequencing**

NGS sequencing was performed using Ion Torrent™ (Life Technologies) technology. This technology uses simple chemistry without fluorophore labeling of nucleotides therefore there is no need of optics and other complicated parts in this instrument. Ion Torrent™ NGS technology uses semiconductor chips where the main part is the flow cell which consists of: micro-machined wells layer; ion or pH sensitive layer and ion sensor layer [25].
The main principle of this technology is the use of H+ released in simple polymerase chain reaction to measure which and how much nucleotides are incorporated in the newly synthesized DNA strand. The nucleotides are flowed in the chip micro-machined wells one by one and each time the nucleotide is incorporated the H+ are released therefore changing the pH of the well. This pH change is detected with ion sensitive layer beneath the well and the signal is transformed in digital form by help of ion sensor layer which is located beneath the ion sensitive layer. If there are two or more identical nucleotides incorporated in the strand the change of pH is larger and therefore proportionally larger signal is transmitted to ion sensor layer. If there is no nucleotide incorporation, no pH changes occur; no signal is transmitted till next nucleotide is added. All information of pH changes in each well is further transmitted to server attached to the instrument where the base calling (sequence writing) is accomplished - the so called "read" is created. There are millions of wells in the flow cell of the chip allowing sequencing millions of ~ 200 bp DNA fragments (reads) in parallel. Acquired reads are then aligned to reference genome leading to each base sequenced many times (coverage) [25]. The *Ion Torrent™ sequenceing platform* require DNA libraries consisting of oligonucleotide mix with ligated platform specific adapters at both ends of oligonucleotides that could be amplified by standard PCR methods using universal primers. The 3' and 5' ends of the DNA library mix were complementary to two universal primers: one used to perform emulsion PCR on glass beads to amplify the signal in each Ion Torrent™ chip well; and the second used to perform sequencing reaction (standard polymerase chain reaction) in each of Ion Torrent™ chip wells. After DNA library preparation all samples were sequenced according to the manufacturer's protocol by combining 10 samples on one 318v2 chip.

**Data analysis**

All the raw sequencing data (reads) were fed to the Torrent Suite 4.4.3 assembler - v4.0.2.1 *plugin* to obtain genome alignment file in bam format. Bam file were intersected with bedtools software to obtain information about positions of interest (30 SNPs) and five nucleotides up- and down-stream from the position of interest. Variant recogniton were performed using Samtools MPileup software (v) and SNPs called with VarScan software (v). Analysing output of VarScan following measures are taken into acount:

- SDP="Raw Read Depth as reported by SAMtools" >=20 or
- DP="Quality Read Depth of bases with Phred score >= 15" >=20 or
- RD="Depth of reference-supporting bases (reads1)" >=1 and
- AD="Depth of variant-supporting bases (reads2)" >=1 and
- FREQ="Variant allele frequency" (0-25%) and
- RBQ="Average quality of reference-supporting bases (qual1)" >=20 and
- ABQ="Average quality of variant-supporting bases (qual2)" >=20

to assess whether there is difference in SNP alleles between mother and fetus.

SNPs meeting criteria stated above were manually reviewed using IGV (v) software to determine mapping quality (MAPQ) of each read and base call quality PHRED score of base in SNP of each read. True calls are used to calculate percentage of reads from fetal DNA. This result is averaged using all eligible loci.

TABLE 2

| Table 2. Example of informative SNPs and FF determination characteristics | | | | | | | | | | | | |
|------|-------|-----------|------------|-------|------|------|-----|-----|----|-------|-------|-------|
| Chr | Name | SNPS | rs | Depth | Type | Geno | % | Fet | N | % | Phred | MapQ |
| chr4 | PCR8 | 137069205 | rs2598548 | 22 | horn | GG | 95 | **A** | 1 | 4,5 | 34 | 58 |
| chr6 | PCR29 | 322255 | rs11242795 | 44 | wt | AA | 93 | **G** | 3 | 6,8 | 26-35 | 64-67 |
| chr6 | PCR13 | 167774024 | rs9347210 | 170 | hom | AA | 94 | **G** | 11 | 6,5 | 28-33 | 4-6 |
| chr4 | PCR15 | 190540979 | rs11132694 | 20 | wt | AA | 95 | **T** | 1 | 5,0 | 25 | 92 |
| chr8 | PCR27 | 110427192 | rs3911483 | 33 | hom | CC | 97 | **T** | 1 | 3,0 5,2 | 31 | 67 |

**REFERENCES**

1. ACOG Practice Bulletin No. 88, December 2007. Invasive prenatal testing for aneuploidy. Obstet Gynecol, 2007. 110(6): p. 1459-67.

2. Elchalal, U., et al., Maternal mortality following diagnostic 2nd-trimester amniocentesis. Fetal Diagn Ther, 2004. 19(2): p. 195-8.

3. Seeds, J.W., Diagnostic mid trimester amniocentesis: how safe? Am J Obstet Gynecol, 2004. 191(2): p. 607-15.

4. Brambati, B., et al., Genetic diagnosis by chorionic villus sampling before 8 gestational weeks: efficiency, reliability, and risks on 317 completed pregnancies. Prenat Diagn, 1992. 12(10): p. 789-99.

5. Firth, H.V., et al., Analysis of limb reduction defects in babies exposed to chorionic villus sampling. Lancet, 1994. 343(8905): p. 1069-71.

6. Ashoor, G., et al., Chromosome-selective sequencing of maternal plasma cell-free DNA for first-trimester detection of trisomy 21 and trisomy 18. Am J Obstet Gynecol. 206(4): p. 322 e1-5.

7. Ashoor, G., et al., Trisomy 13 detection in the first trimester of pregnancy using a chromosome-selective cell-free DNA analysis method. Ultrasound Obstet Gynecol. 41(1): p. 21-5.

8. Bianchi, D.W., et al., Genome-wide fetal aneuploidy detection by maternal plasma DNA sequencing. Obstet Gynecol. 119(5): p. 890-901.

9. Chiu, R.W., et al., Non-invasive prenatal assessment of trisomy 21 by multiplexed maternal plasma DNA sequencing: large scale validity stardy. BMJ. 342: p. c7401.

10. Ehrich, M., et al., Noninvasive detection of fetal trisomy 21 by sequencing of DNA in maternal blood: a study in a clinical setting. Am J Obstet Gynecol. 204(3): p. 205 e1-11.

11. Kazakov, V.I., et al., [Extracellular DNA in the blood of pregnant women]. Tsitologiia, 1995. 37(3): p. 232-6.

12. Lo, Y.M., et al., Presence of fetal DNA in maternal plasma and serum. Lancet, 1997. 350(9076): p. 485-7.

13. Nicolaides, K.H., et al., Validation of targeted sequencing of single-nucleotide polymorphisms for non-invasive prenatal detection of aneuploidy of chromosomes 13, 18, 21, X, and Y. Prenat Diagn. 33(6): p. 575-9.

14. Norton, M.E., et al., Non-Invasive Chromosomal Evaluation (NICE) Study: results of a multicenter prospective cohort study for detection of fetal trisomy 21 and trisomy 18. Am J Obstet Gynecol. 207(2): p. 137 e1-8.

15. Palomaki, G.E., et al., DNA sequencing of maternal plasma reliably identifies trisomy 18 and trisomy 13 as well as Down syndrome: an international collaborative stardy. Genet Med. 14(3): p. 296-305.

16. Palomaki, G.E., et al., DNA sequencing of maternal plasma to detect Down syndrome: an international clinical validation stardy. Genet Med. 13(11): p. 913-20.

17. Samango-Sprouse, C., et al., SNP-based non-invasive prenatal testing detects sex chromosome aneuploidies with high accuracy. Prenat Diagn. 33(7): p. 643-9.

18. Sehnert, A.J., et al., Optimal detection of fetal chromosomal abnormalities by massively parallel DNA sequencing of cell-free fetal DNA from maternal blood. Clin Chem. 57(7): p. 1042-9.

19. Zimmermann, B., et al., Noninvasive prenatal aneuploidy testing of chromosomes 13, 18, 21, X, and Y, using targeted sequencing of polymorphic loci. Prenat Diagn. 32(13): p. 1233-41.

20. Canick, J.A., et al., The impact of maternal plasma DNA fetal fraction on next generation sequencing tests for common fetal aneuploidies. Prenat Diagn. 33(7): p. 667-74.

21. Schlutter, J.M., et al., The cell-free fetal DNA fraction in maternal blood decreases after physical activity. Prenat Diagn. 34(4): p. 341-4.

22. Yu, S.C., et al., Size-based molecular diagnostics using plasma DNA for noninvasive prenatal testing. Proc Natl Acad Sci U S A. 111(23): p. 8583-8.

23. Nygren, A.O., et al., Quantification of fetal DNA by use of methylation-based DNA discrimination. Clin Chem. 56(10): p. 1627-35.

24. Wapner, R.J., et al., Expanding the scope of noninvasive prenatal testing: detection of fetal microdeletion syndromes. Am J Obstet Gynecol. 212(3): p. 332 e1-9.

25. Rothberg, J.M., et al., An integrated semiconductor device enabling non-optical genome sequencing. Nature, 2011. 475(7356): p. 348-52.

**Claims**

1. A method for calculating fetal ccfDNA fraction in maternal plasma comprising detecting the presence of fetus specific alleles (different from maternal alleles) of a set of one or more of single nucleotide polymorphism (SNP) in a target polynucleotide in a subject, wherein the SNP comprises rs10873704 (SEQ IN NO. 1), rs2316921 (SEQ IN NO. 2), rs3991951 (SEQ IN NO. 3), rs13142086 (SEQ IN NO. 4), rs2598548 (SEQ IN NO. 5), rs10155423 (SEQ IN NO. 6), rs7683293 (SEQ IN NO. 7), rs11132694 (SEQ IN NO. 8), rs2287678 (SEQ IN NO. 9), rs888726 (SEQ IN NO. 10), rs7740992 (SEQ IN NO. 11), rs11242795 (SEQ IN NO. 12), rs7756422 (SEQ IN NO. 13), rs6915731 (SEQ IN NO. 14), rs9347210 (SEQ IN NO. 15), rs9691429 (SEQ IN NO. 16), rs4875906 (SEQ IN NO. 17), rs10780162 (SEQ IN NO. 18), rs2005486 (SEQ IN NO. 19), rs3911483 (SEQ IN NO. 20), rs2028902 (SEQ IN NO. 21), rs2904516 (SEQ IN NO. 22), rs2175938 (SEQ IN NO. 23), rs1973118 (SEQ IN NO. 24), rs6498782 (SEQ IN NO. 25), rs3826238 (SEQ IN NO. 26), rs11568105 (SEQ IN NO. 27), rs2139261 (SEQ IN NO. 28), rs4494584 (SEQ IN NO. 29) and rs55969137 (SEQ IN NO. 30), and wherein detecting presence of the SNP in the subject is indicative to calculate fetal ccfDNA fraction in maternal plasma.

2. The method according to claim 1, wherein method comprises detecting the presence of a fetal specific alleles (different from maternal alleles) of a set of one or more single nucleotide polymorphism (SNP) at a position rs10873704 (nucleotide 26 on SEQ IN NO. 1) or any of rs2316921 (nucleotide 26 on SEQ IN NO. 2), rs3991951 (nucleotide 26 on SEQ IN NO. 3), rs13142086 (nucleotide 26 on SEQ IN NO. 4), rs2598548 (nucleotide 26 on SEQ IN NO. 5), rs10155423 (nucleotide 26 on SEQ IN NO. 6), rs7683293 (nucleotide 26 on SEQ IN NO. 7), rs11132694 (nucleotide 26 on SEQ IN NO. 8), rs2287678 (nucleotide 26 on SEQ IN NO. 9), rs888726 (nucleotide 26 on SEQ IN NO. 10), rs7740992 (nucleotide 26 on SEQ IN NO. 11), rs11242795 (nucleotide 26 on SEQ IN NO. 12), rs7756422 (nucleotide 26 on SEQ IN NO. 13), rs6915731 (nucleotide 26 on SEQ IN NO. 14), rs9347210 (nucleotide 26 on SEQ IN NO. 15), rs9691429 (nucleotide 26 on SEQ IN NO. 16), rs4875906 (nucleotide 26 on SEQ IN NO. 17), rs10780162 (nucleotide 26 on SEQ IN NO. 18), rs2005486 (nucleotide 26 on SEQ IN NO. 19), rs3911483 (nucleotide 26 on SEQ IN NO. 20), rs2028902 (nucleotide 26 on SEQ IN NO. 21), rs2904516 (nucleotide 26 on SEQ IN NO. 22), rs2175938 (nucleotide 26 on SEQ IN NO. 23), rs1973118 (nucleotide 26 on SEQ IN NO. 24), rs6498782 (nucleotide 26 on SEQ IN NO. 25), rs3826238 (nucleotide 26 on SEQ IN NO. 26), rs11568105 (nucleotide 26 on SEQ IN NO. 27), rs2139261 (nucleotide 26 on SEQ IN NO. 28), rs4494584 (nucleotide 26 on SEQ IN NO. 29) or rs55969137 (nucleotide 26 on SEQ IN NO. 30).

3. The method according to claim 1 or 2, wherein the presence of any of SNPs is indicative to calculate fetal ccfDNA fraction in maternal plasma.

4. The method according to any of claims 1-3, wherein the method comprises detecting fetal specific allele (different from maternal alleles) in specific genetic variation pattern (SNPs pattern), wherein the SNPs are selected from the group of loci with nucleotide base change consisting of: CHR1, g.85980349 A>G (SEQ ID NO. 1), CHR1, g.104626818 A>G (SEQ ID NO. 2), CHR1, g.224216756 A>G (SEQ ID NO. 3), CHR4, g.126503918 A>T (SEQ ID NO. 4), CHR4, g.137069205 A>G (SEQ ID NO. 5), CHR4, g.170281121 C>G (SEQ ID NO. 6), CHR4, g.174342567 G>T (SEQ ID NO. 7), CHR4, g.190540979 A>T (SEQ ID NO. 8), CHR5, g.102443043 C>T (SEQ ID NO. 9), CHR5, g.175348069 A>G (SEQ ID NO. 10), CHR6, g.261390 A>G (SEQ ID NO. 11), CHR6, g.322255 A>G (SEQ ID NO. 12), CHR6, g.57268764 G>T (SEQ ID NO. 13), CHR6, g.146863404 A>G (SEQ ID NO. 14), CHR6, g.167774024 A>G (SEQ ID NO. 15), CHR7, g.61915623 A>G (SEQ ID NO. 16), CHR8, g.2205101 C>T (SEQ ID NO. 17), CHR8, g.2275622 A>G (SEQ ID NO. 18), CHR8, g.58122242 C>T (SEQ ID NO. 19), CHR8, g.110427192 C>T (SEQ ID NO. 20), CHR11, g.36309909 A>T (SEQ ID NO. 21), CHR12, g.66996166 A>G (SEQ ID NO. 22), CHR15, g.22428943 C>T (SEQ ID NO. 23), CHR15, g.51645910 A>T (SEQ ID NO. 24), CHR16, g.18606396 A>G (SEQ ID NO. 25), CHR16, g.70182024 A>G (SEQ ID NO. 26), CHR17, g.6562461 A>G (SEQ ID NO. 27), CHR17, g.21313222 C>G (SEQ ID NO. 28), CHR17, g.72786911 A>T (SEQ ID NO. 29), CHR22, g.50899756 A>C>G>T (SEQ ID NO. 30), where "g" stands for genomic sequence and "CHR" stands for number of chromosome. Even more in particular, the SNPs may be selected from the variations listed in TABLE 1.

5. An isolated polynucleotide comprising genetic variation at a nucleotide position corresponding to the position of SNPs listed in Table 1.

6. The isolated polynucleotide according to claim 4, wherein the isolated polynucleotide is allele-specific oligonucleotide.

7. A probe for the quantitative detection of SNPs used to calculate fetal ccfDNA fraction in maternal plasma comprising a set of single nucleotide polymorphisms (SNPs) set forth as rs10873704 (SEQ IN NO. 1), rs2316921 (SEQ IN NO. 2), rs3991951 (SEQ IN NO. 3), rs13142086 (SEQ IN NO. 4), rs2598548 (SEQ IN NO. 5), rs10155423 (SEQ IN NO. 6), rs7683293 (SEQ IN NO. 7), rs11132694 (SEQ IN NO. 8), rs2287678 (SEQ IN NO. 9), rs888726 (SEQ IN NO. 10), rs7740992 (SEQ IN NO. 11), rs11242795 (SEQ IN NO. 12), rs7756422 (SEQ IN NO. 13), rs6915731 (SEQ IN NO. 14), rs9347210 (SEQ IN NO. 15), rs9691429 (SEQ IN NO. 16), rs4875906 (SEQ IN NO. 17), rs10780162 (SEQ IN NO. 18), rs2005486 (SEQ IN NO. 19), rs3911483 (SEQ IN NO. 20), rs2028902 (SEQ IN NO. 21), rs2904516 (SEQ IN NO. 22), rs2175938 (SEQ IN NO. 23), rs1973118 (SEQ IN NO. 24), rs6498782 (SEQ IN NO. 25), rs3826238 (SEQ IN NO. 26), rs11568105 (SEQ IN NO. 27), rs2139261 (SEQ IN NO. 28), rs4494584 (SEQ IN NO. 29) and rs55969137 (SEQ IN NO. 30).

8. The probe according to claim 6, which comprises a set of SNPs set for the as rs10873704 (nucleotide 26 on SEQ IN NO. 1) or any of rs2316921 (nucleotide 26 on SEQ IN NO. 2), rs3991951 (nucleotide 26 on SEQ IN NO. 3), rs13142086 (nucleotide 26 on SEQ IN NO. 4), rs2598548 (nucleotide 26 on SEQ IN NO. 5), rs10155423 (nucleotide 26 on SEQ IN NO. 6), rs7683293 (nucleotide 26 on SEQ IN NO. 7), rs11132694 (nucleotide 26 on SEQ IN NO. 8), rs2287678 (nucleotide 26 on SEQ IN NO. 9), rs888726 (nucleotide 26 on SEQ IN NO. 10), rs7740992 (nucleotide 26 on SEQ IN NO. 11), rs11242795 (nucleotide 26 on SEQ IN NO. 12), rs7756422 (nucleotide 26 on SEQ IN NO. 13), rs6915731 (nucleotide 26 on SEQ IN NO. 14), rs9347210 (nucleotide 26 on SEQ IN NO. 15), rs9691429 (nucleotide 26 on SEQ IN NO. 16), rs4875906 (nucleotide 26 on SEQ IN NO. 17), rs10780162 (nucleotide 26 on SEQ IN NO. 18), rs2005486 (nucleotide 26 on SEQ IN NO. 19), rs3911483 (nucleotide 26 on SEQ IN NO. 20), rs2028902 (nucleotide 26 on SEQ IN NO. 21), rs2904516 (nucleotide 26 on SEQ IN NO. 22), rs2175938 (nucleotide 26 on SEQ IN NO. 23), rs1973118 (nucleotide 26 on SEQ IN NO. 24), rs6498782 (nucleotide 26 on SEQ IN NO. 25), rs3826238 (nucleotide 26 on SEQ IN NO. 26), rs11568105 (nucleotide 26 on SEQ IN NO. 27), rs2139261 (nucleotide 26 on SEQ IN NO. 28), rs4494584 (nucleotide 26 on SEQ IN NO. 29) or rs55969137 (nucleotide 26 on SEQ IN NO. 30).

9. A kit for determining fetal ccfDNA fraction in maternal plasma, the kit comprising: (a) at least 30 oligonucleotides that can be used to calculate the fetal ccfDNA fraction in maternal plasma according to any aspect of the present invention wherein the SNP may be selected from the group consisting of: CHR1, g.85980349 A>G (SEQ ID NO. 1), CHR1, g.104626818 A>G (SEQ ID NO. 2), CHR1, g.224216756 A>G (SEQ ID NO. 3), CHR4, g.126503918 A>T (SEQ ID NO. 4), CHR4, g.137069205 A>G (SEQ ID NO. 5), CHR4, g.170281121 C>G (SEQ ID NO. 6), CHR4, g.174342567 G>T (SEQ ID NO. 7), CHR4, g.190540979 A>T (SEQ ID NO. 8), CHR5, g.102443043 C>T (SEQ ID NO. 9), CHR5, g.175348069 A>G (SEQ ID NO. 10), CHR6, g.261390 A>G (SEQ ID NO. 11), CHR6, g.322255 A>G (SEQ ID NO. 12), CHR6, g.57268764 G>T (SEQ ID NO. 13), CHR6, g.146863404 A>G (SEQ ID NO. 14), CHR6, g.167774024 A>G (SEQ ID NO. 15), CHR7, g.61915623 A>G (SEQ ID NO. 16), CHR8, g.2205101 C>T (SEQ ID NO. 17), CHR8, g.2275622 A>G (SEQ ID NO. 18), CHR8, g.58122242 C>T (SEQ ID NO. 19), CHR8, g.110427192 C>T (SEQ ID NO. 20), CHR11, g.36309909 A>T (SEQ ID NO. 21), CHR12, g.66996166 A>G (SEQ ID NO. 22), CHR15, g.22428943 C>T (SEQ ID NO. 23), CHR15, g.51645910 A>T (SEQ ID NO. 24), CHR16, g.18606396 A>G (SEQ ID NO. 25), CHR16, g.70182024 A>G (SEQ ID NO. 26), CHR17, g.6562461 A>G (SEQ ID NO. 27), CHR17, g.21313222 C>G (SEQ ID NO. 28), CHR17, g.72786911 A>T (SEQ ID NO. 29), CHR22, g.50899756 A>C>G>T (SEQ ID NO. 30) and the like; (b) additional elements for example fluorescents for ease of detection of said SNPs and (b) instructions for use; more in particular, the SNP are selected from the SNPs listed in TABLE 1.

Europäisches
Patentamt

European
Patent Office

Office européen
des brevets

# EUROPEAN SEARCH REPORT

Application Number

EP 15 18 2851

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/201507 A1 (RAVA RICHARD P [US] ET AL) 18 August 2011 (2011-08-18) | 1-4,9 | INV.<br>C12Q1/68 |
| A | * the whole document *<br>* para. 9, 27, 221-224 * | 5-8 | |
| X | "Introducing Genome-Wide SNP Array 6.0 Pure performance & Genetic Power",<br>,<br>21 May 2008 (2008-05-21), pages 1-3,<br>XP055239630,<br>Retrieved from the Internet:<br>URL:http://www.genehk.com/news/doc/Genomic s_Genome-Wide Human SNP Array 6.0.pdf<br>[retrieved on 2016-01-08] | 5-9 | |
| A | * the whole document * | 1-4 | |
| X | WO 2015/026967 A1 (NATERA INC [US])<br>26 February 2015 (2015-02-26) | 1-4,9 | |
| A | * the whole document *<br>* p. 2, 24-25 * | 5-8 | |
| X | US 2015/004601 A1 (STRUBLE CRAIG [US] ET AL) 1 January 2015 (2015-01-01) | 1-4,9 | TECHNICAL FIELDS SEARCHED (IPC)<br><br>C12Q |
| A | * the whole document *<br>* para. 8-13 * | 5-8 | |

The present search report has been drawn up for all claims

| Place of search | Date of completion of the search | Examiner |
|---|---|---|
| Munich | 8 January 2016 | Sauer, Tincuta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 18 2851

This annex lists the patent family members relating to the patent documents cited in the above-mentioned European search report.
The members are as contained in the European Patent Office EDP file on
The European Patent Office is in no way liable for these particulars which are merely given for the purpose of information.

08-01-2016

| Patent document cited in search report | | Publication date | Patent family member(s) | | Publication date |
|---|---|---|---|---|---|
| US 2011201507 | A1 | 18-08-2011 | AU | 2010343276 A1 | 06-09-2012 |
| | | | AU | 2010343277 A1 | 06-09-2012 |
| | | | AU | 2010343278 A1 | 06-09-2012 |
| | | | AU | 2010343279 A1 | 06-09-2012 |
| | | | CA | 2785718 A1 | 28-07-2011 |
| | | | CA | 2786351 A1 | 28-07-2011 |
| | | | CA | 2786357 A1 | 28-07-2011 |
| | | | CA | 2786544 A1 | 28-07-2011 |
| | | | DK | 2366031 T3 | 23-02-2015 |
| | | | DK | 2376661 T3 | 02-02-2015 |
| | | | DK | 2513339 T3 | 14-12-2015 |
| | | | EP | 2366031 A1 | 21-09-2011 |
| | | | EP | 2370599 A1 | 05-10-2011 |
| | | | EP | 2376661 A1 | 19-10-2011 |
| | | | EP | 2513339 A1 | 24-10-2012 |
| | | | EP | 2848703 A1 | 18-03-2015 |
| | | | EP | 2848704 A1 | 18-03-2015 |
| | | | EP | 2883965 A1 | 17-06-2015 |
| | | | ES | 2534758 T3 | 28-04-2015 |
| | | | ES | 2534986 T3 | 04-05-2015 |
| | | | GB | 2479080 A | 28-09-2011 |
| | | | GB | 2479471 A | 12-10-2011 |
| | | | GB | 2479476 A | 12-10-2011 |
| | | | GB | 2485644 A | 23-05-2012 |
| | | | GB | 2485645 A | 23-05-2012 |
| | | | HK | 1160185 A1 | 23-11-2012 |
| | | | HK | 1160186 A1 | 30-11-2012 |
| | | | HK | 1162197 A1 | 08-02-2013 |
| | | | HK | 1170269 A1 | 23-08-2013 |
| | | | HK | 1170270 A1 | 02-08-2013 |
| | | | US | 2011201507 A1 | 18-08-2011 |
| | | | US | 2011224087 A1 | 15-09-2011 |
| | | | US | 2011245085 A1 | 06-10-2011 |
| | | | US | 2012094849 A1 | 19-04-2012 |
| | | | US | 2012165203 A1 | 28-06-2012 |
| | | | WO | 2011090557 A1 | 28-07-2011 |
| | | | WO | 2011090558 A1 | 28-07-2011 |
| | | | WO | 2011090559 A1 | 28-07-2011 |
| WO 2015026967 | A1 | 26-02-2015 | NONE | | |
| US 2015004601 | A1 | 01-01-2015 | US | 2015004601 A1 | 01-01-2015 |
| | | | WO | 2014209597 A2 | 31-12-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100376637 A
- US 201261604984 A
- AU 20130903743
- US 201161474362 A
- ES 20100830939

**Non-patent literature cited in the description**

- Invasive prenatal testing for aneuploidy. *Obstet Gynecol, 2007,* December 2007, vol. 110 (6), 1459-67
- **ELCHALAL, U. et al.** Maternal mortality following diagnostic 2nd-trimester amniocentesis. *Fetal Diagn Ther,* 2004, vol. 19 (2), 195-8
- **SEEDS, J.W.** Diagnostic mid trimester amniocentesis: how safe?. *Am J Obstet Gynecol,* 2004, vol. 191 (2), 607-15
- **BRAMBATI, B. et al.** Genetic diagnosis by chorionic villus sampling before 8 gestational weeks: efficiency, reliability, and risks on 317 completed pregnancies. *Prenat Diagn,* 1992, vol. 12 (10), 789-99
- **FIRTH, H.V. et al.** Analysis of limb reduction defects in babies exposed to chorionic villus sampling. *Lancet,* 1994, vol. 343 (8905), 1069-71
- **ASHOOR, G. et al.** Chromosome-selective sequencing of maternal plasma cell-free DNA for first-trimester detection of trisomy 21 and trisomy 18. *Am J Obstet Gynecol.,* vol. 206 (4), 322 e1-5
- **ASHOOR, G. et al.** Trisomy 13 detection in the first trimester of pregnancy using a chromosome-selective cell-free DNA analysis method. *Ultrasound Obstet Gynecol.,* vol. 41 (1), 21-5
- **BIANCHI, D.W. et al.** Genome-wide fetal aneuploidy detection by maternal plasma DNA sequencing. *Obstet Gynecol.,* vol. 119 (5), 890-901
- **CHIU, R.W. et al.** Non-invasive prenatal assessment of trisomy 21 by multiplexed maternal plasma DNA sequencing: large scale validity stardy. *BMJ.,* vol. 342, c7401
- **EHRICH, M. et al.** Noninvasive detection of fetal trisomy 21 by sequencing of DNA in maternal blood: a study in a clinical setting. *Am J Obstet Gynecol.,* vol. 204 (3), 205 e1-11
- **KAZAKOV, V.I. et al.** Extracellular DNA in the blood of pregnant women. *Tsitologiia,* 1995, vol. 37 (3), 232-6
- **LO, Y.M. et al.** Presence of fetal DNA in maternal plasma and serum. *Lancet,* 1997, vol. 350 (9076), 485-7
- **NICOLAIDES, K.H. et al.** Validation of targeted sequencing of single-nucleotide polymorphisms for non-invasive prenatal detection of aneuploidy of chromosomes 13, 18, 21, X, and Y. *Prenat Diagn.,* vol. 33 (6), 575-9
- **NORTON, M.E. et al.** Non-Invasive Chromosomal Evaluation (NICE) Study: results of a multicenter prospective cohort study for detection of fetal trisomy 21 and trisomy 18. *Am J Obstet Gynecol.,* vol. 207 (2), 137 e1-8
- **PALOMAKI, G.E. et al.** DNA sequencing of maternal plasma reliably identifies trisomy 18 and trisomy 13 as well as Down syndrome: an international collaborative stardy. *Genet Med.,* vol. 14 (3), 296-305
- **PALOMAKI, G.E. et al.** DNA sequencing of maternal plasma to detect Down syndrome: an international clinical validation stardy. *Genet Med.,* vol. 13 (11), 913-20
- **SAMANGO-SPROUSE, C. et al.** SNP-based non-invasive prenatal testing detects sex chromosome aneuploidies with high accuracy. *Prenat Diagn.,* vol. 33 (7), 643-9
- **SEHNERT, A.J. et al.** Optimal detection of fetal chromosomal abnormalities by massively parallel DNA sequencing of cell-free fetal DNA from maternal blood. *Clin Chem.,* vol. 57 (7), 1042-9
- **ZIMMERMANN, B. et al.** Noninvasive prenatal aneuploidy testing of chromosomes 13, 18, 21, X, and Y, using targeted sequencing of polymorphic loci. *Prenat Diagn.,* vol. 32 (13), 1233-41
- **CANICK, J.A. et al.** The impact of maternal plasma DNA fetal fraction on next generation sequencing tests for common fetal aneuploidies. *Prenat Diagn.,* vol. 33 (7), 667-74
- **SCHLUTTER, J.M. et al.** The cell-free fetal DNA fraction in maternal blood decreases after physical activity. *Prenat Diagn.,* vol. 34 (4), 341-4
- **YU, S.C. et al.** Size-based molecular diagnostics using plasma DNA for noninvasive prenatal testing. *Proc Natl Acad Sci U S A.,* vol. 111 (23), 8583-8

- **NYGREN, A.O. et al.** Quantification of fetal DNA by use of methylation-based DNA discrimination. *Clin Chem.*, vol. 56 (10), 1627-35
- **WAPNER, R.J. et al.** Expanding the scope of noninvasive prenatal testing: detection of fetal microdeletion syndromes. *Am J Obstet Gynecol.*, vol. 212 (3), 332 e1-9
- **ROTHBERG, J.M. et al.** An integrated semiconductor device enabling non-optical genome sequencing. *Nature,* 2011, vol. 475 (7356), 348-52

SEQUENCE LISTING

<110>  LATVIAN BIOMEDICAL RESEARCH AND STUDY CENTRE

<120>  SET OF OLIGONUCLEOTIDES AND METHOD FOR DETECTION OF FETAL DNA
FRACTION IN MATERNAL PLASMA

<130>  EPP2015/14

<160>  11

<170>  PatentIn version 3.5


<210>  1
<211>  52
<212>  DNA
<213>  Artificial sequence

<400>  1
cttttctttc acttttttgg gtatt[a/g]cta tggactaatg aatttttatg ta            52


<210>  2
<211>  52
<212>  DNA
<213>  Artificial sequence

<400>  2
agatagatag gcagccaaca aaagt[a/g]tac atacacacac agtgaataag ca            52

<210>  3
<211>  52
<212>  DNA
<213>  Artificial sequence

<400>  3
acaatgcagc cctttgctt cctaa[a/g]ctc accagttccc tcgctccacc tc             52


<210>  4
<211>  52
<212>  DNA
<213>  Artificial sequence

<400>  4
cttatttcag tttagtgcag atgaa[a/t]ttt tttttttttt ttatggaggt ca           52


<210>  3
<211>  52
<212>  DNA
<213>  Artificial sequence

<400>  5
gcaggtgggt agttgaagtg tgtgt[a/g]tgt gtgtgtgtgt gttttatttc ct           52


<210>  3
<211>  52
<212>  DNA
<213>  Artificial sequence

<400> 6
aagcaaaaag aaaaccaatc aaaac[c/g]cta cactttaact ttgttcccct ga          52


<210>   3
<211>   52
<212>   DNA
<213>   Artificial sequence

<400>   7
tttctctgtt ttggtttagt ttgtt[g/t]ggg ggggggttgat ttgttgcatt tt          52


<210>   3
<211>   52
<212>   DNA
<213>   Artificial sequence

<400>   8
caagaggctt ccagtgctcc ccaga[a/t]ggg ttttgacatc ttatcattgt tt          52


<210>   3
<211>   52
<212>   DNA
<213>   Artificial sequence

<400>   9
aaaataatgt tttatcatca ctgta[c/t]ttt tgcaagcaaa cagctctctc ag          52


<210>   3
<211>   52
<212>   DNA
<213>   Artificial sequence

<400>   10
agattgacct tacatggttg cctgg[a/g]aga tccaatgaga tcagtacgaa ca          52


<210>   3
<211>   52
<212>   DNA
<213>   Artificial sequence

<400>   11
tcaaaatatc tcatgagagt ggtgc[a/g]tac aattaatgaa cctaccttga ca          52


<210>   3
<211>   52
<212>   DNA
<213>   Artificial sequence

<400>   12
gggaatgatg gtggggccat cctcc[a/g]gcg tcttgctttt atgtggtgtt at          52


<210>   3
<211>   52
<212>   DNA
<213>   Artificial sequence

<400>   13

ggtttgcaga tggctgtctt ctcat[g/t]gta tcttcaaatg gttgagagca ga          52


<210>    3
<211>    52
<212>    DNA
<213>    Artificial sequence

<400>    14
ccaccactgt ttccacgtat tccgt[a/g]tgt gtgtgtgtgt gtgtgtgtgt gt          52


<210>    3
<211>    52
<212>    DNA
<213>    Artificial sequence

<400>    15
taaatttata tttccaaacc aggac[a/g]ctt gtgtcactta gctgcttaaa cc          52


<210>    3
<211>    52
<212>    DNA
<213>    Artificial sequence

<400>    16
tgagaaaatt ctttgtgacg agtgc[a/g]ttc aactcacaga gttaaatttc tt          52


<210>    3
<211>    52
<212>    DNA
<213>    Artificial sequence

<400>    17
aaatcaagca tggtcataac tgttt[c/t]agc agcatttttc agtcgtgcgt ac          52


<210>    3
<211>    52
<212>    DNA
<213>    Artificial sequence

<400>    18
tcttggtgaa tactccattc gacac[a/g]tta aaagaatgtg tattcttttg tt          52


<210>    3
<211>    52
<212>    DNA
<213>    Artificial sequence

<400>    19
aagggagaaa tttccagccc ccttg[c/t]ggc agctagctga aaaacaggct cc          52


<210>    3
<211>    52
<212>    DNA
<213>    Artificial sequence

<400>    20

```
cctgttttct acatcatccc aacat[c/t]att attattatta ttattattat ta          52


<210>   3
<211>   52
<212>   DNA
<213>   Artificial sequence


<400>   21
actcaaccct cttcacctgg tgttt[a/t]aaa aaaaaaaaaa agagagagga ag          52


<210>   3
<211>   52
<212>   DNA
<213>   Artificial sequence


<400>   22
taaaatgatg tgtaactaca atcac[a/g]ggg gccccgcata atttaaagaa at          52


<210>   3
<211>   52
<212>   DNA
<213>   Artificial sequence


<400>   23
tctcatgcct cctggtgtct ctggg[c/t]gat caatgtggcc ttaagatgct cg          52


<210>   3
<211>   52
<212>   DNA
<213>   Artificial sequence


<400>   24
tgtggttttt tttttttttt ttttt[a/t]atc aaatccgagga ctgttctga ta          52


<210>   3
<211>   52
<212>   DNA
<213>   Artificial sequence


<400>   25
aagacttgct aagtggagaa tttct[a/g]tgt ccagtcattc ctggatcact tc          52


<210>   3
<211>   52
<212>   DNA
<213>   Artificial sequence


<400>   26
gtagcaggca caatgaggac aaggc[a/g]agt gtcttctctg ggggaatcag ca          52


<210>   3
<211>   52
<212>   DNA
<213>   Artificial sequence


<400>   27
```

```
caagatggaa tggttgatag aaaca[a/g]ggt caaatgaaga aatgtgactg gc          52
```

```
<210>  3
<211>  52
<212>  DNA
<213>  Artificial sequence
```

```
<400>  28
agagactcag ctgacctctg tccac[c/g]aag ctaacccatg gccctcaaac at          52
```

```
<210>  3
<211>  52
<212>  DNA
<213>  Artificial sequence
```

```
<400>  29
agcaggagca tctctgaatt ccctt[a/t]aaa aaaaaaaaaa aaaaagact gt          52
```

```
<210>  3
<211>  52
<212>  DNA
<213>  Artificial sequence
```

```
<400>  30
cagagtcaga agcaaggaag gtaag[a/c/g/t]ggg gggggtccc agaatctcag gg          54
```